# EUROPEAN PATENT APPLICATION

(11) **EP 4 764 483 A1**
(43) Date of publication of application: **24.06.2026**
(21) Application number: 24222031.7
(22) Date of filing: 20.12.2024
(51) Int. Cl.: G01N 27/327, G01N 33/543, G01F 1/64, G01F 1/68

(54) **AN ELECTROCHEMICAL SENSOR ARRANGEMENT AND A METHOD FOR MEASURING A PROPERTY OF A FLUID**

(71) Applicant: Stichting IMEC Nederland, 5656 AE Eindhoven (NL)
(72) Inventor: VERSCHUEREN, Alwin, 5221 LC 's-Hertogenbosch (NL)
(74) Representative: AWA Sweden AB

(57) **Abstract**

An electrochemical sensor arrangement (100) for measuring a property of a fluid comprises: a first electrode (110) and a second electrode (120) arranged on a first surface (132) of a first layer (130); and readout circuitry (140) connected to the first electrode (110) and the second electrode (120), wherein the readout circuitry (140) is formed at a second surface (134) of the first layer (130) opposite to the first surface (132) or at a substrate (142) separate from the first layer (130); wherein a second shape of the second electrode (120) completely encloses the first shape of the first electrode (110) with a spacing (114) being defined between the first electrode (110) and the second electrode (120); and wherein a first connection (136) between the first electrode (110) and the readout circuitry (140) comprises a first via (112) extending from the first electrode (110) through the first layer (130).

## Description

### Technical field

The present description relates to electrochemical sensors that measure a property of a fluid. In particular, the present description relates to an electrochemical sensor arrangement and a method for measuring a property of a fluid.

### Background

Electrochemical sensors may be used for measuring a property of a fluid. The electrochemical sensors may for instance be used for measuring electrical conductivity, temperature, flow rate or concentration of an analyte in the fluid.

The electrochemical sensors use at least two electrodes that both make contact with the fluid. Each electrode has an associated electrical potential and electric field lines will penetrate the fluid in contact with the electrodes. The electric field lines will extend in the fluid from one electrode to the other electrode.

A path of the electric field lines through the fluid affects the electrochemical measurements. Design of the electrodes may define the path of the electric field lines through the fluid. Thus, there is a need for proper design of the electrodes for ensuring accurate measurements by electrochemical sensors.

### Summary

An objective of the present description is to enable accurate measurements of a property of a fluid. A particular objective of the present description is to provide an electrochemical sensor arrangement designed to provide accurate measurements.

These and other objectives are at least partly met by the invention as defined in the independent claims. Preferred embodiments are set out in the dependent claims.

According to a first aspect, there is provided an electrochemical sensor arrangement for measuring a property of a fluid, said electrochemical sensor arrangement comprising: a first electrode and a second electrode arranged on a first surface of a first layer, wherein the first electrode and the second electrode are configured to make contact with the fluid for measuring the property of the fluid; and readout circuitry connected to the first electrode and the second electrode for reading out signals from the first and second electrodes, wherein the readout circuitry is formed at a second surface of the first layer opposite to the first surface or at a substrate separate from the first layer; wherein the first electrode has a first shape on the first surface and the second electrode has a second shape on the first surface, wherein the second shape of the second electrode defines an opening within the second electrode such that the second shape of the second electrode completely encloses the first shape of the first electrode with a spacing being defined between the first electrode and the second electrode; and wherein a first connection between the first electrode and the readout circuitry comprises a first via extending from the first shape of the first electrode through the first layer.

Thanks to the second electrode being arranged to completely enclose the first electrode, the electrochemical sensor arrangement allows electric field lines to be formed in a well-controlled and easily predictable manner in the fluid. In particular, a relation between the first electrode and the second electrode may be similar or equal across an entire area of the first and second electrodes. This implies that fringing fields in the fluid may be avoided. The avoiding of fringing fields may ensure that accurate measurements by the electrochemical sensor may be performed. The avoiding of fringing fields may be particularly important for providing accurate determination of electrolyte conductivity in the fluid.

Each of the electrodes further need to be electrically connected to readout circuitry for enabling measurements to be read out. However, since the second shape of the second electrode fully encloses the first shape of the first electrode, the electrical connection to the first electrode could affect the electric field lines in the fluid. Thanks to the first connection comprising a first via extending through the first layer on which the first and second electrode are arranged, the electrical connection is provided without affecting the electric field lines in the fluid.

Thus, the first via is provided through the first layer. This implies that the first via provides access to a connection to the first electrode at a side of the first layer opposite to a side at which the first electrode is arranged. This allows a flexibility of providing the readout circuitry connected to the first via and the readout circuitry may be connected to the first via in various manners, as will be described herein.

It should be realized that a second electrical connection between the second electrode and the readout circuitry will not need to be carefully provided for avoiding that the second electrical connection affects the electric field lines in the fluid. Rather, since the second electrode encloses the first electrode, the second electrical connection may for instance be configured to extend along the first surface of the first layer, away from the first electrode.

As used herein, electrochemical sensor arrangement relates to any sensor arrangement wherein signals, such as electrical potentials, are provided to a first and a second electrode and an electrical response is measured. The electrical response may for instance be an electrical current, potential or charge. The electrical response is representative of a property of a fluid making contact with the first and second electrodes.

The fluid may be any fluid on which measurements are to be made. The fluid may be in a liquid state and may herein also be referred to as a liquid.

The first electrode and the second electrode are formed by an electrically conductive material. The electrodes may be formed by a same material but may alternatively be formed by different materials. Each electrode is configured to make contact with the fluid, such that the electrodes form electrical interfaces to the fluid.

Each of the first electrode and the second electrode may be formed as a thin-film structure on the first layer. Thus, the electrodes may be formed on the first surface of the first layer by deposition of a thin film on the first surface. This may provide a simple manner of manufacturing the electrodes. However, it should be realized that the electrodes may be formed in another manner.

The first layer may comprise an electrically insulating material. The electrically insulating material may prevent electrical connection between the first and second electrodes through the electrically insulating material. The first layer may further comprise a conducting material for forming via(s) through the electrically insulating material. The first layer may further comprise semiconducting material for forming transistors at the second surface of the first layer.

The first surface of the first layer may be planar, such that the first electrode and the second electrode arranged on the first surface may be arranged in a common plane. However, it should be realized that the first surface need not be planar throughout an extension of the first surface. In particular, outside an area in which the first and the second electrode are arranged, the first surface may be curved or bent. A shape of the first surface may be adapted to an application in which the electrochemical sensor arrangement is to be used.

The first layer is configured to allow the fluid to make contact with the first surface. Thus, the first surface may form part of a channel through which the fluid is transported or a container in which the fluid is arranged. Thus, the first layer may form part of a wall defining a space in which the fluid is present. The first layer may alternatively be configured to be inserted into the fluid for allowing the first surface to make contact with the fluid.

The first layer may at least in some embodiments further provide a barrier to the fluid such that the fluid is not allowed to make contact with the readout circuitry. The readout circuitry may be protected from making contact with the fluid by the first layer forming part of a wall with the first surface arranged to face the fluid. The readout circuitry may further be protected from making contact with the fluid by being arranged in an enclosure forming a protection at the second surface of the first layer.

The readout circuitry may be any appropriate circuitry for reading signals from electrodes. The readout circuitry may comprise suitable electrical components for reading out signals from the electrodes. For instance, the readout circuitry may be configured to acquire analog signals from the electrodes.

The readout circuitry may also be configured to perform analog-to-digital conversion of acquired signals. This may facilitate further processing of the acquired signals and may also facilitate transfer of the signals to a separate processing unit. In some embodiments, the readout circuitry may further be configured to process the signals.

The readout circuitry may be arranged at the second surface of the first layer. This implies that the readout circuitry may be arranged on the second surface or in the first layer at the surface. For instance, the first layer may be formed by a substrate in which an integrated circuit may be formed. Then, the readout circuitry may be formed at least partly in the first layer at the second surface by defining semiconductor components in the first layer.

According to an embodiment, the readout circuitry may thus be arranged at a frontside of a substrate for forming an integrated circuit. The first layer may be formed by the substrate and the second surface of the first layer may be the frontside of the substrate. The first surface of the first layer may be a backside of the substrate and the electrodes may thus be arranged on the backside of the substrate.

According to another embodiment, the first layer may be formed on top of a readout circuitry with the second surface of the first layer being arranged facing the readout circuitry. Thus, the readout circuitry may be arranged at the second surface of the first layer. In this case, the readout circuitry may be formed on a separate substrate, i.e., the forming of the readout circuitry does not take place on the first layer. Rather, the first layer may be formed on the separate substrate once the readout circuitry has been formed. In such case, the first layer may form part of back-end-of-line (BEOL) layers.

The readout circuitry being formed at a substrate separate from the first layer may also imply that the substrate is separate from the first layer when the readout circuitry is formed. Thus, when the readout circuitry has been formed, the separate substrate may be attached to or integrated with the first layer. The separate substrate may for instance be bonded to the first layer.

It should be realized that the substrate being separate from the first layer does not necessarily imply that, in the electrochemical sensor arrangement, the first layer is physically separated from the substrate. Rather, the substrate is a separate part, i.e., a different part, of the electrochemical sensor arrangement. Thus, the first layer may be attached to the substrate.

However, it should be further realized that the separate substrate is not necessarily arranged in a location beneath the electrodes. Thus, the separate substrate need not be arranged at a projection of the electrodes onto the second surface of the first layer. Rather, the first connection may allow a connection between the readout circuitry and the first via to have an extension in a direction parallel to the area of the first and the second electrodes. This may allow the readout circuitry to be displace sideways from the electrodes, which may be useful in some embodiments, e.g., if an acceptable thickness of the electrochemical sensor arrangement is limited.

The first electrode may have any shape on the first surface. The second electrode further has a second shape which fully encloses the first shape. Thus, the second electrode is arranged around the entire first shape of the first electrode. Since there is further a spacing between the first electrode and the second electrode, the second shape of the second electrode defines an opening in which the first electrode is formed. The opening may thus be defined by an inner perimeter of the second shape.

The first electrode may for instance have a disc shape and the second electrode may have a ring shape. This may provide a simple shape of the electrodes which may facilitate manufacturing of the electrochemical sensor arrangement. However, it should be realized that the first shape and the second shape may have other forms.

It should further be realized that a shape of the second electrode outside the inner perimeter, which defines the opening, may not be very important. Thus, the second electrode may have a square shape with a circular opening within the square shape defined by the inner perimeter of the second electrode. The second electrode may even extend around additional electrodes having a respective opening associated with each of the first electrode and the additional electrode(s).

As mentioned above, the first via may be formed by a conductive material. The first via may thus be formed a conductive material extending through an insulating material in the first layer. The insulating material arranged around the first via may ensure that the first via is insulated from other vias in the first layer and from electrodes arranged on the first surface of the first layer.

The first via is configured to extend through the first layer. Thus, the first via may extend entirely through the first layer between the first surface and the opposite second surface of the first layer. The first via may thus provide an electrical connection between the electrodes arranged on the first surface of the first layer and a readout circuitry which is arranged at the second surface or connected to the second surface of the first layer.

It should be realized that the first via may comprise a plurality of staggered via parts. For instance, the first layer may be a multi-layer, wherein layers in the multi-layer are formed sequentially on top of each other with via parts formed through the layers and being connected to each other in a staggered arrangement. Thus, the first via may not necessarily extend along a straight line between the first surface and the second surface of the first layer. The first layer may for instance be formed by multiple BEOL layers through which the via parts are formed.

However, according to another embodiment, the first via may be arranged along a straight line between the first surface and the second surface of the first layer. The first via may have a constant cross-section through the first layer.

According to an embodiment, an outer border of the opening defined by the second shape of the second electrode has a corresponding shape to the first shape of the first electrode such that the spacing between the first electrode and the second electrode is equidistant across the first shape of the first electrode. The spacing is defined for any given point on the outer border of the first shape of the first electrode as the distance to the closest point on the second shape of the second electrode.

An inner perimeter of the second shape of the second electrode may define the outer border of the opening.

Thanks to the spacing between the first electrode and the second electrode is equidistant across the first shape of the first electrode, electric field lines have equal strength around the first electrode. This removes a spatial dependence of electric field strength along a circumference of the first shape of the first electrode. This allows for very high accuracy in determination of fluid properties and may be particularly prominent in determination of electrolyte conductivity in the fluid.

It should be realized that corresponding shapes of the first and second shape may not necessarily mean that an outer perimeter of the first shape is identically scaled in all directions to an inner perimeter of the second shape. Rather, the second shape of the second electrode corresponds to the first electrode such that an equidistant spacing between the first electrode and the second electrode is provided around a circumference of the first electrode.

According to an embodiment, the outer border of the opening and an outer border of the first shape form concentric circles. In other words, an inner perimeter of the second shape and an outer perimeter of the first shape may form concentric circles. These shapes of the first and second electrodes is a particular manner of ensuring that an equidistant spacing between the first electrode and the second electrode is provided around a circumference of the first electrode.

The use of concentric circles may facilitate simple manufacturing of the electrochemical sensor arrangement. This also ensures that all electric field lines between the first electrode and the second electrode in the fluid will have a perfect rotational symmetry.

According to an embodiment, the electrochemical sensor arrangement comprises a second connection between the second electrode and the readout circuitry, and the second connection comprises a second via extending from the second shape of the second electrode through the first layer.

This may facilitate connection of the readout circuitry to the first electrode and the second electrode. The first and second vias may provide contact points at a common interface surface.

According to an embodiment, the read-out circuitry is formed at the substrate separate from the first layer, wherein the first connection includes a contact point formed at the second surface of the first layer, wherein the first via is configured to extend from the first shape of the first electrode to the contact point, wherein the first connection is further configured to connect the contact point and the readout circuitry being arranged at the substrate being bonded to the first layer.

This implies that the substrate carrying the readout circuitry may be manufactured separately from an electrode arrangement. This may ensure that manufacturing steps of one part may not affect the other part, which may simplify manufacturing of the substrate with the readout circuitry and the electrode arrangement, respectively.

The electrode arrangement may thus be manufactured to form the first layer with via(s) and electrodes arranged on the first surface of the first layer. The substrate may be bonded to the first layer for forming a compact electrochemical sensor arrangement.

The substrate carrying the readout circuitry may form an integrated circuit. However, the substrate carrying the readout circuitry may form a carrier on which an integrated circuit is mounted. For instance, the substrate may be a printed circuit board.

The readout circuitry may be connected to the contact point via one or more electrical connections, which may extend in a direction perpendicular to a plane of the second surface of the first layer and/or a direction parallel to the plane of the second surface.

It should also be realized that the first via may extend from the first electrode to the contact point along a straight line or along a non-straight line, e.g., if the first via comprises staggered via parts.

According to an embodiment, the first layer is a substrate formed by silicon or glass.

Thus, the substrate is arranged between the electrodes and the readout circuitry. This may facilitate manufacturing of the electrochemical sensor arrangement.

The substrate may be formed by silicon or glass. This may facilitate manufacturing of the electrochemical sensor arrangement in facilities for mass-volume semiconductor processing. However, it should be realized that, in other embodiments, the substrate may instead, for instance, be formed by quartz, sapphire, silicon carbide, gallium arsenide, or germanium.

According to an embodiment, the first via at the first surface of the first layer has a smaller cross-section than the first shape of the first electrode and the first via is fully covered by the first shape of the first electrode.

This ensures that the first via is not in contact with the fluid. This implies that a choice of material of the first via need not be selected to withstand contact with the fluid. Thus, the first via need not be formed by a noble metal and that, for instance, the first via may be formed by copper.

According to an embodiment, the first via has a length of at least 20 µm, such as at least 100 µm, extending through the first layer.

Correspondingly, a thickness of the first layer between the first surface and the second surface, through which thickness the first via extends, may be at least 20 µm, such as at least 100 µm.

This may imply that a parasitic capacitance between the first and second electrodes, via first and second connections to the first and second electrodes, respectively, and via the readout circuitry, is low compared to a direct capacitance between the first and the second electrode via the fluid. This ensures that measurements will not be affected by the parasitic capacitance.

If the first layer is formed by a substrate, the length of the first via may typically be at least 100 µm, such as at least 300 µm.

According to an embodiment, at least a part of the first shape of the first electrode is provided with a bioreceptor configured to interact with an analyte in the fluid.

The first electrode may thus be functionalized providing a function of interacting with the analyte in the fluid. This implies that a target analyte in the fluid may be situated at the first electrode facilitating accurate detection of a property of the fluid. This may be used, in particular, for determining a concentration of the target analyte.

The bioreceptor may have a binding affinity for a specific analyte. Thus, the bioreceptor may be configured to bind the target analyte. the bioreceptor may for instance be an aptamer or an antibody configured to bind the target analyte. However, the bioreceptor may not necessarily be configured to bind the target analyte but may rather interact with the target analyte. The bioreceptor may for instance be an enzyme or an ionophore configured to interact with the target analyte.

It should be realized that the first electrode may be provided with one or more bioreceptors. Thus, the first electrode may be provided with more than one bioreceptor configured to interact with different analytes in the fluid.

In addition or alternatively, the second electrode may also be functionalized. The at least part of the second shape of the second electrode may be provided with a bioreceptor configured to interact with an analyte in the fluid. If both the first and the second electrodes are provided with bioreceptors, the first and the second electrodes may be provided with the same bioreceptor and/or with different bioreceptors.

The entire part of the first shape of the first electrode may be provided with the bioreceptor. Thus, the entire first electrode may provide a functionalized surface. However, it should be realized that the bioreceptor may be configured to cover only part of the first shape of the first electrode.

According to an embodiment, the electrochemical sensor arrangement further comprises a reference electrode arranged on the first surface of the first layer.

Thus, the electrochemical sensor arrangement may be configured to perform measurements based on three electrodes. This may facilitate providing a linear response between the property of the fluid, such as a concentration of an analyte, and a measured signal. For instance, the electrochemical sensor arrangement may use a potentiostat electronic circuit.

According to an embodiment, a connection between the reference electrode and the readout circuitry comprises a reference via extending from the reference electrode through the first layer.

Thus, when the electrochemical sensor arrangement comprises a reference electrode, the electrochemical sensor arrangement may further comprise a reference via. This may facilitate connection of the readout circuitry to the reference electrode, in addition to the connections to the first electrode and the second electrode. Each of the reference via, the first via, and the second via may provide contact points at a common interface surface.

According to an embodiment, the electrochemical sensor arrangement further comprises an additional electrode, wherein the first electrode and the additional electrode are configured for performing separate measurements of the property of the fluid, wherein the second electrode is configured to provide a shared counter electrode to the first electrode and the additional electrode, wherein the second shape of the second electrode defines separate openings for the first electrode and the additional electrode within the second electrode such that the second shape of the second electrode further completely encloses a shape of the additional electrode with a spacing being defined between the additional electrode and the second electrode.

This facilitates scaling of a number of electrode pairs provided in the electrochemical sensor arrangement for multiplexed sensing. Since the second electrode may act as a shared counter electrode, electrode pairs may be arranged very close to each other. It should be realized that an electrode pair is defined by a first electrode acting as a working electrode and a second electrode acting as a counter electrode. In this respect, different electrode pairs may share the counter electrode such that the pairs may be arranged close to each other.

Thanks to the second electrode being arranged to completely enclose the first electrode, the second electrode may further also completely enclose the additional electrode in a simple arrangement of the second electrode.

However, it should be realized that the electrochemical sensor arrangement may in another embodiment be provided with a plurality of electrode pairs, wherein the electrodes of the electrode pairs are separate from each other. Thus, each electrode pair may comprise a first electrode and a second electrode, which are separate from first and second electrodes of other electrode pairs.

According to an embodiment, the first electrode and the second electrode form a first pair of electrodes and the electrochemical sensor arrangement further comprises: a second pair of electrodes comprising an additional first electrode and an additional second electrode, wherein a shape of the additional second electrode defines an opening within the additional second electrode such that the shape of the additional second electrode completely encloses a shape of the additional first electrode with a spacing being defined between the additional first electrode and the additional second electrode, wherein an additional first connection between the additional first electrode and the readout circuitry comprises an additional first via extending from the shape of the additional first electrode through the first layer; and a heater arranged between the first pair of electrodes and the second pair of electrodes; wherein the first pair of electrodes is configured to perform a first measurement of the property of the fluid and the second pair of electrodes is configured to perform a second measurement of the property of the fluid, wherein a difference between the first measurement and the second measurement is representative of mass flow of the fluid.

Thus, separate measurements of a property of the fluid may be performed by two different pairs of electrodes. For instance, the pairs of electrodes may be configured to measure fluid conductivity, which may be strongly dependent on temperature of the fluid. Thanks to using a heater arranged between the first pair of electrodes and the second pair of electrodes, a temperature of the fluid may be changed between the first pair of electrodes and the second pair of electrodes. This implies that the fluid conductivity measured at a respective location of the first and second pairs of electrodes may strongly depend on temperature of the fluid at the respective location, which in turn depends on the mass flow of the fluid between the location of the first and second pairs of electrodes passing a location of the heater. This provides a very accurate measurement of mass flow.

The heater may be a resistive heater. This may provide a simple manner of implementing the heater. The resistive heater may in fact share electrical connections with the second electrode and the additional second electrode. This implies that a simple connection of circuitry for controlling the heater and for reading out signals from the pairs of electrodes may be provided.

The first and second pair of electrodes may be formed by separate electrodes. Thus, the second electrode of the first pair may be separate from the additional second electrode of the second pair. However, according to an embodiment, the second electrode and the additional second electrode may be provided by a shared counter electrode, as described above.

According to an embodiment, the heater is arranged on a thermally insulating material.

The heater may be arranged on the first surface of the first layer facing the fluid. The electrochemical sensor arrangement may further comprise a thermally insulating material, such that the heater is configured to be arranged facing the fluid in a first direction from the heater with the thermally insulating material being arranged in a second direction from the heater, the second direction being opposite to the first direction.

The thermally insulating material may ensure that a heat flux from the heater is predominantly used for heating the fluid and not heating the first layer. This may ensure that the heater is efficiently used and may improve accuracy and sensitivity of a mass flow sensor.

According to a second aspect, there is provided a method for measuring a property of a fluid, said method comprising: arranging a first electrode and a second electrode in contact with the fluid, wherein the first electrode and the second electrode are arranged on a first surface of a first layer, wherein the first electrode has a first shape on the surface and the second electrode has a second shape on the surface, wherein the second shape of the second electrode defines an opening within the second electrode such that the second shape of the second electrode completely encloses the first shape of the first electrode with a spacing being defined between the first electrode and the second electrode; and reading out a signal from the first and second electrodes by a readout circuitry, wherein the readout circuitry is formed at a second surface of the first layer opposite to the first surface or at a substrate separate from the first layer, and wherein a first connection between the first electrode and the readout circuitry comprises a first via extending from the first shape of the first electrode through the first layer.

Effects and features of this second aspect are largely analogous to those described above in connection with the first aspect. Embodiments mentioned in relation to the first aspect are largely compatible with the second aspect.

The method facilitates that fringing fields in the fluid may be avoided. The avoiding of fringing fields may ensure that accurate measurements of the property of the fluid may be performed. The avoiding of fringing fields may be particularly important for providing accurate determination of electrolyte conductivity in the fluid.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Figs 1a-c are schematic perspective, top and cross-sectional views, respectively, of an electrochemical sensor arrangement according to an embodiment.
Figs 2a-h are schematic view of different options of an architecture of the electrochemical sensor arrangement.
Figs 3a-c are schematic perspective, top and cross-sectional views, respectively, of an electrochemical sensor arrangement according to another embodiment.
Figs 4a-c are schematic perspective, top and cross-sectional views, respectively, of an electrochemical sensor arrangement according to yet another embodiment.
Fig. 5 is a perspective view of an electrochemical sensor arrangement according to yet another embodiment.
Fig. 6 is a perspective view of an electrochemical sensor arrangement according to yet another embodiment.
Fig. 7 is a flow chart of a method according to an embodiment.

### Detailed description

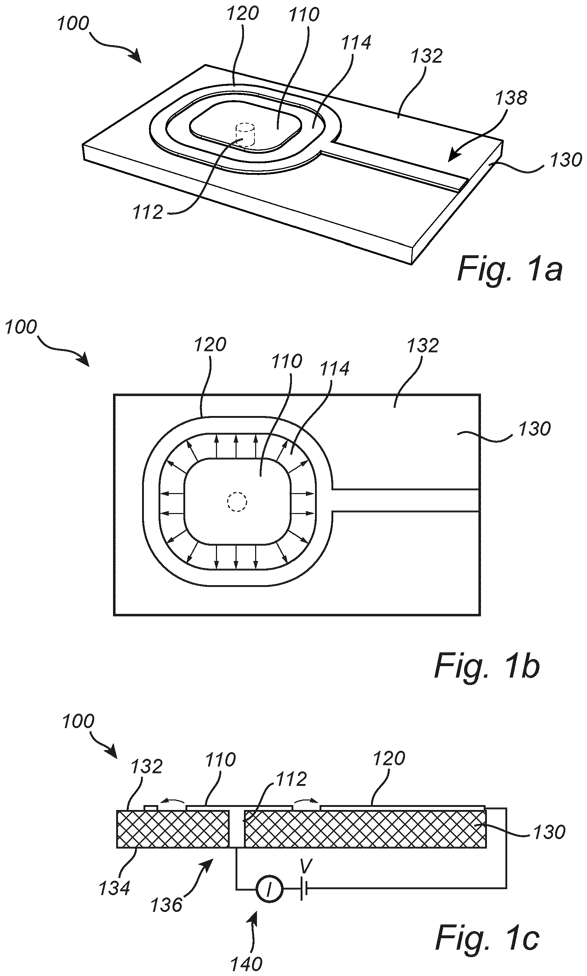

Figs 1a-c illustrates an electrochemical sensor arrangement 100 according to a first embodiment. The electrochemical sensor arrangement 100 is configured to measure a property of a fluid. Fig. 1a shows a perspective view of the electrochemical sensor arrangement 100. Fig. 1b shows a top view of the electrochemical sensor arrangement 100. Fig. 1c shows a cross-sectional view of the electrochemical sensor arrangement 100 with a schematic indication of readout circuitry.

The electrochemical sensor arrangement 100 comprises a first electrode 110 and a second electrode 120 arranged on a first layer 130. The first electrode 110 and the second electrode 120 may each be formed by a conductive material for providing an electrical interface to the fluid.

The electrochemical sensor arrangement 100 may be configured to be arranged in relation to the fluid such that the first electrode 110 and the second electrode 120 make contact with the fluid. The first layer 130 may for instance form part of a wall of a space in which the fluid is arranged, such as a wall of a container or a wall of a channel through which the fluid is transported.

The first electrode 110 and the second electrode 120 may comprise a conductive material which is suitable for making contact with the fluid. Thus, according to an embodiment, each of the first electrode 110 and the second electrode 120 may comprise a noble metal, such as gold, platinum, or silver, facing the fluid. This may ensure that the electrodes 110, 120 are suited for making contact with the fluid. The first electrode 110 and the second electrode 120 may alternatively comprise carbon or a conductive polymer, such as polyaniline (PANI), or poly(3,4-ethylenedioxythiophene)-poly(styrenesulfonate) (PEDOT:PSS).

The first electrode 110 and the second electrode 120 are arranged on a first surface 132 of the first layer 130. The electrochemical sensor arrangement 100 may be configured such that the first surface 132 of the first layer 130 may be arranged for facing the fluid. The first layer 130 may further have a thickness having a second surface 134 opposite to the first surface 132. The electrochemical sensor arrangement 100 may be configured such that the second surface 134 does not make contact with the fluid.

The first electrode 110 has a first shape on the first surface 132 and the second electrode 120 has a second shape on the first surface 132. The second shape of the second electrode 120 defines an opening within the second electrode 120 such that the second shape of the second electrode 120 completely encloses the first shape of the first electrode 110 with a spacing 114 being defined between the first electrode 110 and the second electrode 120. This facilitates providing equal electric field strength in the fluid around the shape of the first electrode 110, as illustrated in Fig. 1b. Hence, fringing fields may be reduced or completely avoided ensuring that accurate determinations of the property of the fluid may be provided based on measurements by the electrochemical sensor arrangement 100.

An outer border of the opening defined by the second shape of the second electrode 120 has a corresponding shape to the first shape of the first electrode 110 such that the spacing 114 between the first electrode 110 and the second electrode 120 is equidistant across the first shape of the first electrode 110.

It should be realized that the first electrode 110 may have various shapes. The first electrode 110 may be arranged to fill an area defined by an outer border. The second electrode 120 may have a corresponding shape to the outer border of the first shape such that an equidistant spacing 114 may be defined. The second electrode 120 may thus have an inner border defining an outer border of the opening in which the first electrode 110 is arranged. The second electrode 120 may have a freely defined shape outside the inner border.

It should be realized that the spacing 114 being equidistant across the first shape of the first electrode 110 may ensure that fringing fields are avoided in the fluid. However, it should be realized that the spacing 114 may not be exactly equidistant across the first shape. Rather, some variation of a size of the spacing 114 may be present, for instance in view of variations within tolerances of manufacturing of the electrochemical sensor arrangement 100.

The electrochemical sensor arrangement further comprises a first via 112 extending from the first shape of the first electrode 110 through the first layer 130. The first via 112 may thus extend through a thickness of the first layer 130 from the first surface 132 of the first layer 130 to the second surface 134 of the first layer 130. The first via 112 may ensure that an electrical connection may be provided to the first electrode 110 even though the first shape of the first electrode 110 is completely enclosed by the second shape of the second electrode 120. The electrical connection is further provided without affecting the electric field lines in the fluid.

The first via 112 at the first surface 132 of the first layer may have a smaller cross-section than the first shape of the first electrode 110. The first via 112 may further be fully covered by the first shape of the first electrode 110. This implies that the first via 112 may not make contact with the fluid. Hence, the first via 112 may be formed from a conductive material which may be freely chosen without need of being suitable for making contact with the fluid. The first via 112 may be formed by a non-noble metal. For instance, the first via 112 may be formed by copper, tungsten, aluminum, graphite, an epoxy filled with nanoparticles, such as silver nanoparticles, or a conductive polymer, such as PANI or PEDOT:PSS.

The first layer 130 may be configured to provide an insulating material. The insulating material may prevent electrical connection through the first layer 130 between the first electrode 110 and the second electrode 120. The insulating material may also be arranged around the first via 112 for providing insulation around the first via 112. The first layer 130 may be formed by a homogeneous material through which the first via 112 and possibly other vias as discussed below are arranged.

However, the first layer 130 may also or additionally be provided with insulating materials forming an insulating sheath around the first via 112 and at the first surface 132 on which the first electrode 110 and the second electrode 120 are arranged. The first layer 130 may for instance be formed by silicon and may further be provided with insulating material at the first surface 132 and around the first via 112 and any further vias in the first layer 130.

The first layer 130 may thus comprise insulating materials, such as silicon oxide, (borosilicate) glass, quartz, diamond, aluminum oxide, sapphire, magnesium oxide, titanium oxide, zirconium oxide, silicon carbide, silicon nitride, ceramics.

The first layer 130 may also or alternatively comprise polymer materials, which may be flexible. This may facilitate providing a flexible first layer 130 that may be bent or conformed to a desired shape. The first layer 130 may thus for instance comprise polyethylene terephthalate (PET), polyethylene naphthalate (PEN), polycarbonate (PC), polyethersulfone (PES), polyarylate (PAR) and polyimide (PI).

The electrochemical sensor arrangement 100 further comprises readout circuitry 140 connected to the first electrode 110 and the second electrode 120. The readout circuitry 140 may be separated from the first electrode 110 and the second electrode 120 by the first layer 130. The readout circuitry 140 may be arranged at the second surface 134 of the first layer 130 or at a separate substrate from the first layer 130.

The readout circuitry 140 may be connected by a first connection 136 to the first electrode 110. The first connection 136 includes the first via 112 extending from the first shape of the first electrode 110 through the first layer 130. This allows the first connection 136 to extend through the first layer 130 such that the first electrode 110 is connected to the readout circuitry 140 without affecting electric field lines in the fluid between the first electrode 110 and the second electrode 120.

The readout circuitry 140 may be connected by a second connection 138 to the second electrode 120. Since the second shape of the second electrode 120 encloses the first shape of the first electrode 110, the second connection 138 may extend along the first surface 132 of the first layer 130 and need not extend through the first layer 130. Rather, the second connection 138 may extend around the first layer 130 for providing a connection to the readout circuitry 140, as schematically illustrated in Fig. 1c.

The electrochemical sensor arrangement 100 may be configured to provide an electrical signal to the first electrode 110 and the second electrode 120 and may be configured to detect an electrical response signal, which may be representative of a property of the fluid. For instance, the electrochemical sensor arrangement 100 may be configured to apply electrical potentials to the first electrode 110 and the second electrode 120 and to measure an electrical response signal. The electrical response signal may for instance be an electrical current.

The electrochemical sensor arrangement 100 may thus be configured to measure any property of the fluid, to which the electrical response signal may be related. For instance, the electrochemical sensor arrangement 100 may be configured to measure an electrical response signal representative of a concentration of an analyte in the fluid.

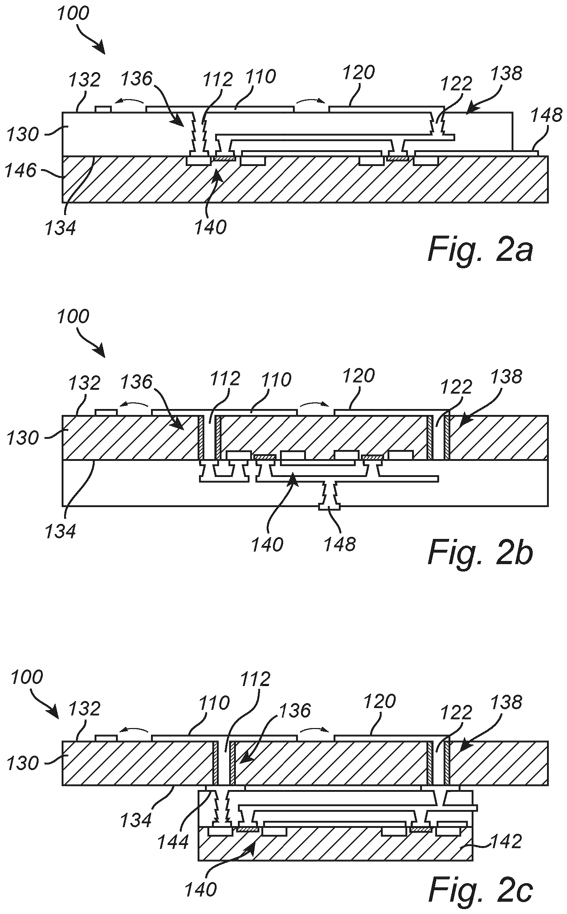

Referring now to Figs 2a-h, various options for an architecture of the electrochemical sensor arrangement 100 will be discussed.Figs 2a-h show different architectures for providing connections between the first electrode 110 and the second electrode 120 to the readout circuitry 140.

In the options illustrated in Figs 2a-d, the electrochemical sensor arrangement 100 includes a silicon substrate. In options illustrated in Figs 2eh, the electrochemical sensor arrangement 100 includes a glass substrate, such as borosilicate glass. It should however be realized that substrates of other materials may be used, as indicated above.

Fig. 2a shows an option, wherein the readout circuitry 140 is formed as an integrated circuit on the silicon substrate 146. The readout circuitry 140 may for instance be formed by complementary metal-oxide-semiconductor (CMOS) electronics integrated with the silicon substrate 146.

The first layer 130 may be formed integrated on the silicon substrate 146. The first layer 130 may thus for instance comprise silicon oxide or silicon nitride deposited on the silicon substrate 146. The first via 112 of the first connection 136 and a second via 122 of the second connection 138 may also be formed extending through the first layer 130. Thus, the first layer 130 may be formed as back-end-of-line (BEOL) layers integrated on the silicon substrate 146. It should be realized that the first via 112 and the second via 122 may be staggered through the first layer 130 and do not necessarily follow a straight path.

The readout circuitry 140 may further be provided with a contact pad 148 at a surface of the substrate 146. The contact pad 148 may be arranged laterally displaced from the readout circuitry 140 along the substrate 146. The contact pad 148 may thus not be covered by the first layer 130 to allow access to the contact pad 148. The contact pad 148 may allow another circuitry to be connected to the readout circuitry 140, e.g., for providing further processing of measurements acquired by the readout circuitry 140 or supply power.

The first layer 130 may thus be monolithically integrated at a frontside of the substrate 146 comprising the readout circuitry 140. The readout circuitry 140 may thus be arranged at the second surface 134 of the first layer 130 being integrated on the substrate carrying the readout circuitry 140.

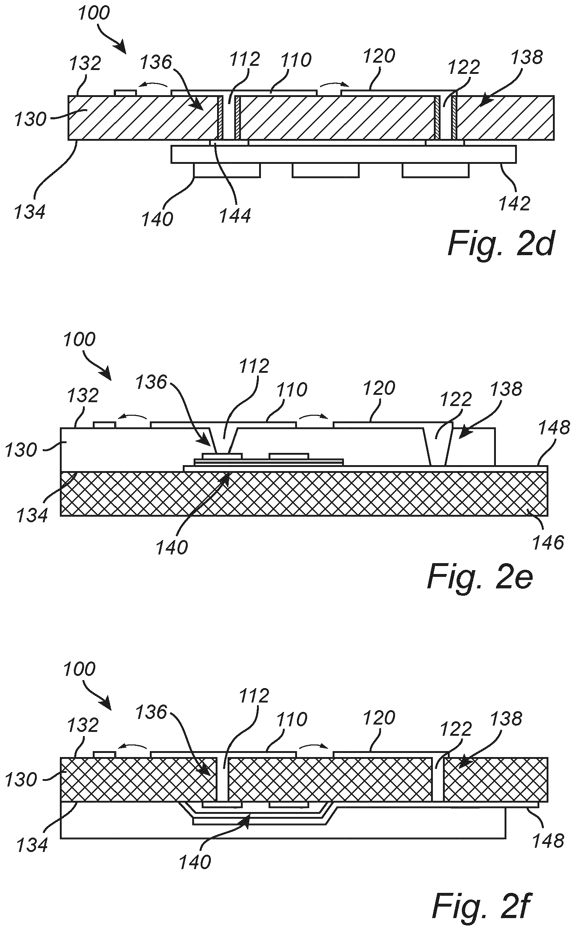

Fig. 2e shows an option similar to the option in Fig. 2a. The first layer 130 is here also monolithically integrated at a frontside of the substrate 146 comprising the readout circuitry 140. The readout circuitry 140 may thus be arranged at the second surface 134 of the first layer 130 being integrated on the substrate carrying the readout circuitry 140. However, in Fig. 2e, the readout circuitry 140 is formed by thin-film transistors arranged on the substrate 146.

The first layer 130 may be formed integrated on the substrate 146 on the thin-film electronics. The first layer 130 may thus for instance comprise silicon oxide or silicon nitride deposited on the substrate 146. The first via 112 of the first connection 136 and the second via 122 of the second connection 138 may also be formed extending through the first layer 130.

The contact pad 148 may be arranged laterally displaced from the readout circuitry 140 along the substrate 146.

Fig. 2b shows another option, wherein the readout circuitry 140 is formed as an integrated circuit on the silicon substrate. The readout circuitry 140 may be formed by CMOS electronics integrated with the silicon substrate.

However, in this option, the first electrode 110 and the second electrode 120 are arranged at an opposite side of the silicon substrate. Thus, in the option in Fig. 2b the silicon substrate forms the first layer 130 with the first electrode 110 and the second electrode 120 arranged integrated at a backside to the electronic circuitry for providing the readout circuitry 140.

The readout circuitry 140 is thus integrated at the second surface 134 of the first layer 130.

The first via 112 and the second via 122 may be formed through the substrate forming the first layer 130. The first via 112 and the second via 122 may thus be formed as through-silicon vias, which may be provided with an insulating material around the vias for insulating the vias 112, 122 from the silicon substrate.

The electrochemical sensor arrangement 100 may further be provided with BEOL layers at the second surface 134 of the first layer 130 and may further be provided with a contact pad 148 at an opposite side of the BEOL layers to the readout circuitry 140.

Fig. 2f shows an option similar to the option in Fig. 2b. The first layer 130 is here also formed by a substrate having the readout circuitry 140 monolithically integrated at the second surface 134 of the substrate. The first electrode 110 and the second electrode 120 are arranged at an opposite side of the substrate to the readout circuitry 140 for being integrated at a backside to the electronic circuitry. In Fig. 2f, like in Fig. 2e, the readout circuitry 140 is formed by thin-film transistors arranged on the substrate.

The first via 112 and the second via 122 may be formed through the substrate forming the first layer 130. The first via 112 and the second via 122 may thus be formed as through-glass vias.

The thin-film electronic circuitry may be protected by a dielectric material. The contact pad 148 may be arranged at the second surface 134 of the substrate forming the first layer 130, laterally displaced from the readout circuitry 140 along the substrate.

Fig. 2c shows an option, wherein the readout circuitry 140 is formed on a substrate 142 separate from the first layer 130. Thus, the first layer 130 may be formed by a silicon substrate separate from the substrate 142 on which the readout circuitry 140 is formed.

The first electrode 110 and the second electrode 120 are arranged at the first surface 132 of the first layer 130. The first layer 130 may further be provided with through-silicon vias forming the first via 112 and the second via 122, which may be provided with an insulating material around the vias for insulating the vias 112, 122 from the silicon substrate.

The first layer 130 may further be provided with contact points at the second surface 134 of the first layer 130. Thus, the first connection 136 includes a contact point 144 at the second surface 134 of the first layer 130. The first via 112 is configured to extend from the first shape of the first electrode 110 to the contact point 144.

The readout circuitry 140 may in this case be formed as an application-specific integrated circuit (ASIC) that is arranged in a chip. The chip may be bonded to the second surface 134 of the first layer 130 with the readout circuitry 140 being connected to the contact points at the second surface 134 of the first layer 130. Thus, the first connection 136 may further be configured to connect the contact point 144 and the readout circuitry 140 being arranged at the substrate 142 bonded to the first layer 130. Similar connections are provided for the second connection between the second electrode 120 and the readout circuitry 140.

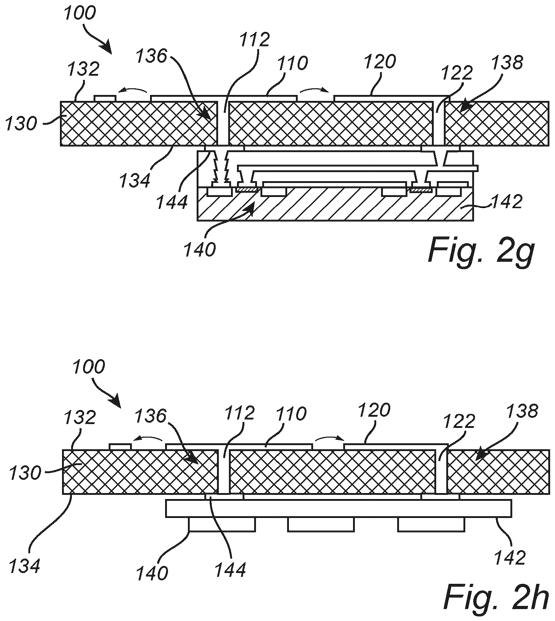

Fig. 2g shows an option similar to the option in Fig. 2c. The readout circuitry 140 is here also formed on a substrate 142 separate from the first layer 130. In the option in Fig. 2g, the first layer 130 may be formed by a glass substrate separate from the substrate 142 on which the readout circuitry 140 is formed.

The first electrode 110 and the second electrode 120 are arranged at the first surface 132 of the first layer 130. The first layer 130 may further be provided with through-glass vias forming the first via 112 and the second via 122.

The first layer 130 may further be provided with contact points at the second surface 134 of the first layer 130, similar to the contact points shown in the option in Fig. 2c.

The readout circuitry 140 may in this case also be formed as an ASIC that is arranged in a chip. Similar to the option in Fig. 2c, the chip may be bonded to the second surface 134 of the first layer 130 with the readout circuitry 140 being connected to the contact points at the second surface 134 of the first layer 130.

Fig. 2d shows another option similar to the option in Fig. 2c. The readout circuitry 140 is here also formed on a substrate 142 separate from the first layer 130. In the option in Fig. 2d, like in the option in Fig. 2c, the first layer 130 may be formed by a silicon substrate separate from the substrate 142 on which the readout circuitry 140 is formed.

The first electrode 110, the second electrode 120, the first via 112 and the second via 122 may be provided in a similar manner to the arrangement in the option in Fig. 2c. The first layer 130 may further be provided with contact points at the second surface 134 of the first layer 130, similar to the contact points shown in the option in Fig. 2c.

The readout circuitry 140 may in this case be formed by electronic components arranged on a printed circuit board, forming a separate substrate 142 to the substrate of the first layer 130. The printed circuit board 142 may be bonded to the second surface 134 of the first layer 130 with the readout circuitry 140 being connected to the contact points at the second surface 134 of the first layer 130.

Fig. 2h shows an option similar to the option in Fig. 2g. The readout circuitry 140 is here also formed on a substrate 142 separate from the first layer 130. In the option in Fig. 2h, like in the option in Fig. 2g, the first layer 130 may be formed by a glass substrate separate from the substrate 142 on which the readout circuitry 140 is formed.

The first electrode 110, the second electrode 120, the first via 112 and the second via 122 may be provided in a similar manner to the arrangement in the option in Fig. 2g. The first layer 130 may further be provided with contact points at the second surface 134 of the first layer 130, similar to the contact points shown in the option in Fig. 2g.

The readout circuitry 140 may in this case, similar to the option shown in Fig. 2d, also be formed by electronic components arranged on a printed circuit board, forming a separate substrate 142 to the substrate of the first layer 130. The printed circuit board 142 may be bonded to the second surface 134 of the first layer 130 with the readout circuitry 140 being connected to the contact points at the second surface 134 of the first layer 130.

In the options where the readout circuitry 140 is arranged on a separate substrate 142 that is not monolithically integrated with the first layer 130, the separate substrate 142 with the readout circuitry 140 may be arranged in other relations to the first layer 130. The separate substrate 142 need not be bonded to the first layer 130 and may for instance be arranged displaced from the first layer 130 in a direction along a plane of the first layer 130. The first electrical connection 136 and the second electrical connection 138 may thus further comprise wires or other connections extending from the contact points at the second surface 134 of the first layer 130 to the readout circuitry 140 at the separate substrate 142.

As described above, the first via 112 and the second via 122 may extend through the first layer 130. The thickness of the first layer 130 may depend on the option used for the electrochemical sensor arrangement 100.

For instance, the first via 112 may have a length of at least 20 µm. This may be a relevant length when the first via 112 extends through a layer monolithically integrated at a frontside of the substrate 146 comprising the readout circuitry 140, as shown in the options of Figs 2a and 2e.

According to an alternative, the first via 112 may have a length of at least 100 µm. This may be a relevant length when the first electrode 110 and the second electrode 120 are arranged integrated at a backside to the readout circuitry 140, as shown in the options of Figs 2b and 2f. The substrate forming the first layer 130 may in these cases typically have a thickness of more than 300 µm, such as 375 µm, such that the first via 112 would have a corresponding length being at least 300 µm. However, the thickness of the substrate forming the first layer 130 may be reduced by backgrinding, such that in some cases, the first via 112 may have a length as small as 100 µm.

According to yet another alternative, the first via 112 may have a length of at least 100 µm. This may be relevant when the first layer 130 is separate from the substrate 142. In such cases, the first layer 130 may need to have a thickness of at least 300 µm, in order to avoid the first layer 130 being fragile.

It should be realized that, even though the architectures shown in Fig. 2a-h include a second via 122 extending through the first layer 130, the second connection 138 between the second electrode 120 and the readout circuitry 140 may be provided in different manners and need not necessarily include the second via 122 extending through the first layer 130.

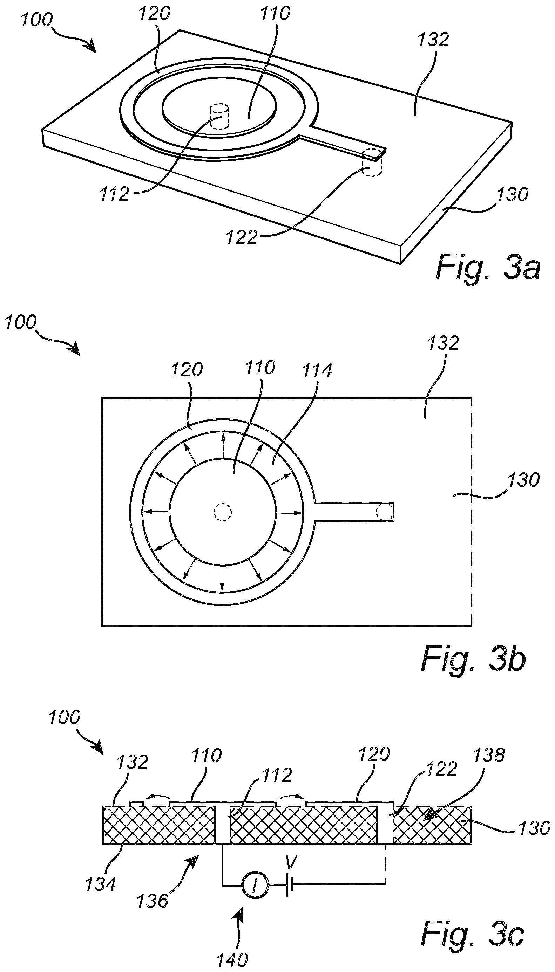

Referring now to Figs 3a-c, a version of the electrochemical sensor arrangement 100 according to an embodiment is shown. Fig. 3a shows a perspective view of the electrochemical sensor arrangement 100, Fig. 3b shows a top view of the electrochemical sensor arrangement 100, and Fig. 3c shows a cross-sectional view of the electrochemical sensor arrangement 100 with a schematic indication of readout circuitry.

The first electrode 110 of the electrochemical sensor arrangement 100 in Figs 3a-c has a first shape corresponding to a disk. In addition, an outer border of the opening in the second shape of the second electrode 120 has a circular shape. In other words, an inner perimeter of the second shape of the second electrode 120 defining the spacing 114 has a circular shape.

Thus, the spacing 114 between the first electrode 110 and the second electrode 120 may be ring-shaped with an outer border of the opening in the second shape of the second electrode 120 and an outer border of the first shape of the first electrode 110 forming concentric circles.

This geometry ensures that all electric field lines between the first electrode 110 and the second electrode 120 have a perfect rotational symmetry, as illustrated in Fig. 3b. The rotational symmetry of the electric field lines removes a spatial dependence of the electric field lines along a circumference of the first shape of the first electrode 110. This allows for excellent accuracy in determination of the property of the fluid from measurements by the electrodes.

The electrochemical sensor arrangement 100 in Figs 3a-c further comprises a second via 122 extending through the first layer 130. It should be realized that the second via 122 may be used in any embodiment and is not linked to any particular shape of the second electrode 120.

Thus, the second connection 138 between the second electrode 120 and the readout circuitry 140 may comprise a second via 122 extending from the second shape of the second electrode 120 through the first layer 130. This may imply that the second via 122 provides a connection to a same interface as the first via 112. The second via 122 may be formed in the same manner as described above for the first via 112.

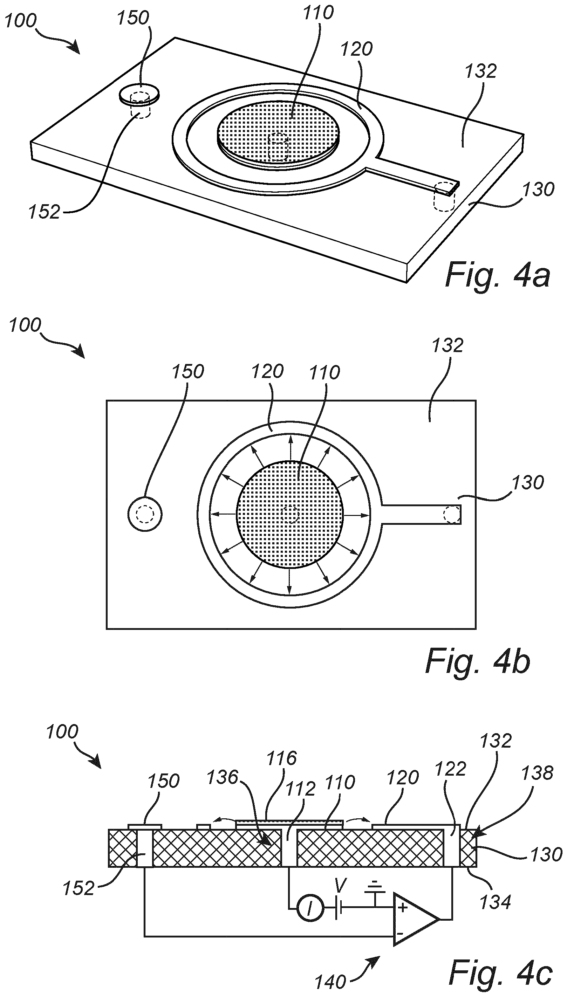

Referring now to Figs 4a-c, a version of the electrochemical sensor arrangement 100 according to another embodiment is shown. Fig. 4a shows a perspective view of the electrochemical sensor arrangement 100, Fig. 4b shows a top view of the electrochemical sensor arrangement 100, and Fig. 4c shows a cross-sectional view of the electrochemical sensor arrangement 100 with a schematic indication of readout circuitry.

The electrochemical sensor arrangement shown in Figs 4a-c further comprises a reference electrode 150 arranged on the first surface 132 of the first layer 130. A connection between the reference electrode 150 and the readout circuitry 140 further comprises a reference via 152 extending from the reference electrode 150 through the first layer 130. The reference via 152 may be formed in a corresponding manner to the first via 112 described above.

However, it should be realized that the reference electrode 150 need not necessarily be connected to the readout circuitry 140 using the reference via 152.

The electrochemical sensor arrangement including the reference electrode 150 may use three-electrode electrochemical measurement techniques.

The reference electrode 150 may be fabricated as a non-polarizable reference electrode, for instance using silver/silver chloride (Ag/AgCl) materials. If furthermore the electrical current of the reference electrode 150 is minimized, for example by connecting the reference electrode 150 to a high-ohmic input of an operational amplifier, then the reference electrode 150 will maintain the same electrical potential as a bulk of the fluid. This implies that the applied voltage drop (between the first electrode 110 and the reference electrode 150) will be fully absorbed between the first electrode (acting as a working electrode) and the bulk fluid. All resulting electrical current will flow between first (working) electrode 110 and the second electrode 120 (acting as a counter electrode).

Using for example the potentiostat electronic circuit 140 illustrated in Fig. 4c, a voltage of the second (counter) electrode 120 will automatically be controlled in order to drain the electrical current. For example, with a first (working) electrode 110 of platinum, and a fixed potential of about -0.4V between the first (working) electrode and the reference electrode 150 of Ag/AgCl, the resulting electrical current will depend linearly on available dissolved oxygen level in the fluid. Oxygen molecules will be reduced to OH⁻ ions at the first (working) electrode 110 and will be transported to the second (counter) electrode 120, where the OH⁻ ions will be converted back to oxygen.

Using a rotationally symmetric electrode structure with a disk-shaped first electrode 110 enclosed by a ring-shaped opening of the second electrode 120, the electrical currents (and associated ions) will flow evenly in all directions between the first (working) electrode 110 and the second (counter) electrode 120. This ensures that the concentrations of all electrochemical species are as uniform as possible, to yield high reliability and reproducibility of the electrochemical measurements, and the properties of the fluid determined from the measurements.

The electrochemical sensor arrangement 100 in Figs 4a-c further comprises a functionalized first (working) electrode 110. It should be realized that the functionalized electrode may be used in any embodiment and is not linked to any particular shape of the second electrode 120 or to the use of a reference electrode 150.

At least a part of the first shape of the first electrode 110 may be provided with a bioreceptor 116 configured to interact with an analyte in the fluid. This implies that the first electrode 110 may be functionalized.

The bioreceptor may be, for instance, an enzyme (like glucose oxidase for glucose analyte sensing), or an ionophore membrane (like valinomycin for potassium analyte sensing), or an aptamer with redox probe (like methylene blue, for many types of analytes).

The bioreceptor functionalization will determine the working electrode current (depending on the applied voltage, and the concentration of the target analyte), and this current is balanced by the counter electrode current to sustain a steady-state condition. Again, the benefit of a perfect rotational symmetry between working and counter electrode is that the current density (and associated ion and analyte species transport) can be sustained evenly, yielding high reliability and reproducibility of the electrochemical measurements, and the analyte concentrations determined from the measurements.

It should be further realized that also the second electrode 120 or at least part of the second electrode 120 may be provided with a bioreceptor.

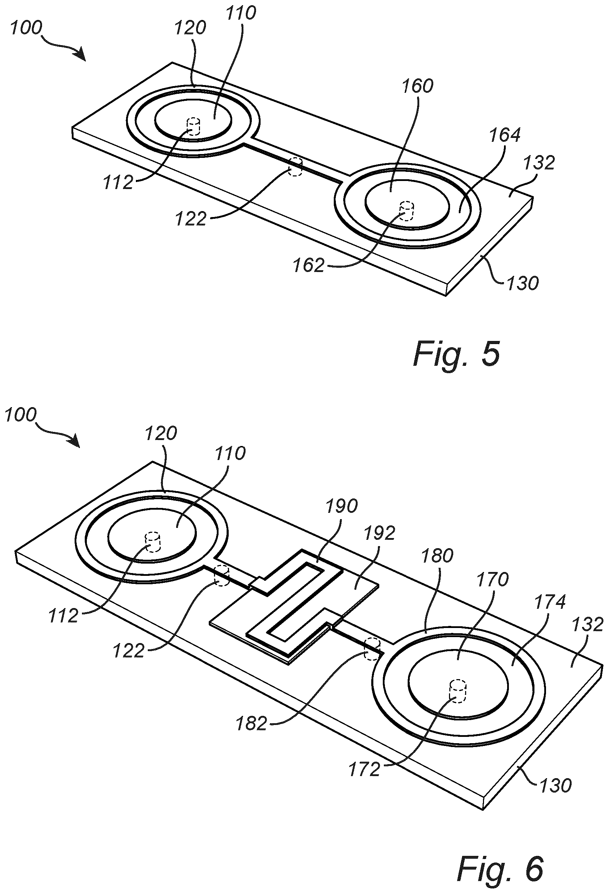

Referring now to Fig. 5, a version of the electrochemical sensor arrangement 100 according to another embodiment is shown. Fig. 5 shows a perspective view of the electrochemical sensor arrangement 100.

The electrochemical sensor arrangement 100 shown in Fig. 5 further comprises an additional electrode 160 arranged on the first surface 132 of the first layer 130. The first electrode 110 and the additional electrode 160 are configured for performing separate measurements of the property of the fluid. The additional electrode 160 may be formed in the same manner as the first electrode 110 as described above.

The second electrode 120 is configured to provide a shared counter electrode to the first electrode 110 and the additional electrode 160. Thus, the second shape of the second electrode 120 is configured to completely enclose the first shape of the first electrode 110 and the shape of the additional electrode 160. The second shape of the second electrode 120 thus defines separate openings for the first electrode 110 and the additional electrode 160 within the second shape of the second electrode 120.

Thus, the second shape of the second electrode 120 further completely encloses a shape of the additional electrode 160 with a spacing 164 being defined between the additional electrode 160 and the second electrode 120.

The electrochemical sensor arrangement 100 may further comprise an additional via 162 extending from the shape of the additional electrode 160 through the first layer 130 for providing a connection to the readout circuitry 140. The additional via 162 may be formed in a corresponding manner to the first via 112.

The electrochemical sensor arrangement 100 may thus provide a plurality of electrode pairs. In Fig. 5, a first electrode pair is formed by the first electrode 110 and the second electrode 120 and a second electrode pair is formed by the additional electrode 160 and the second electrode 120.

The second electrode 120 may be shared by multiple working electrodes for providing a shared counter electrode to the multiple working electrodes. Portions of the second electrode 120 associated with respective working electrodes may be connected in any manner. For instance, as shown in Fig. 5, the second shape of the second electrode 120 may comprise two ring shapes associated with the first electrode 110 and the additional electrode 160, respectively, with the two ring shapes connected by a connecting portion. However, according to an alternative, the second shape of the second electrode 120 may for instance have a rectangular shape defining an area around the first electrode 110 and the additional electrode 160 with circular openings formed in the rectangular shape around the first electrode 110 and the additional electrode 160, respectively.

The use of a shared second electrode 120 is beneficial for scaling up the number of electrode pairs for multiplexed sensing by the electrochemical sensor arrangement 100. This may be used, for example, for measuring local variations in analyte concentration or fluid temperature, or use functionalized working electrodes with different bioreceptors for multiparameter sensing. By the second electrode 120 being interconnected and associated with all working electrodes, and by routing a connection to the second electrode 120 with a single via 122 through the first layer 130, a total number of N working electrodes can be independently measured using only N+1 conductive vias through the first layer 130.

Referring now to Fig. 6, use of the electrochemical sensor arrangement 100 as a mass flow sensor will be described.

The electrochemical sensor arrangement 100 comprises a first pair of electrodes and a second pair of electrodes. Thus, the first electrode 110 and the second electrode 120 according to any of the above-described embodiments form a first pair of electrodes.

The electrochemical sensor arrangement further comprises an additional first electrode 170 and an additional second electrode 180 being formed in a corresponding manner to the first electrode and the second electrode according to any one of the embodiments described above. In particular, a shape of the additional second electrode 180 defines an opening within the additional second electrode 180 such that the shape of the additional second electrode 180 completely encloses a shape of the additional first electrode 170 with a spacing 174 being defined between the additional first electrode 170 and the additional second electrode 180. Further, an additional first connection between the additional first electrode 170 and the readout circuitry 140 comprises an additional first via 172 extending from the shape of the additional first electrode 170 through the first layer 130. As further shown in Fig. 6, an additional second connection between the additional second electrode 180 and the readout circuitry 140 may also comprise an additional second via 182 extending from the shape of the additional second electrode 180 through the first layer 130.

The electrochemical sensor arrangement 100 further comprises a heater 190 arranged between the first pair of electrodes and the second pair of electrodes. The heater 190 may be a resistive heater. However, it should be realized that another type of heater may be used. For instance, the heater may be a microwave heater, an infrared heater. It should also be realized that even though the term heater 190 is used, the heater 190 may be configured to control temperature of a fluid such that the temperature is not necessarily increased by instead be decreased. Thus, as a further alternative, a Peltier cooler may be used.

Fig. 6 illustrates using a resistive heater 190. The resistive heater 190 may be configured to share connections to electronic circuitry with the second electrodes 120, 180. Thus, an electrical current may be passed through the resistive heater 190 by using the second via 122 and the additional second via 182 as leads of the resistive heater 190.

Electrical current is passed through the resistive heater 190 to increase the temperature of the fluid in proximity of the heater 190. The first pair of electrodes is configured to perform a first measurement of electrical current (resistance) between the electrodes of the first pair and the second pair of electrodes is configured to perform a second measurement of electrical current (resistance) between the electrodes of the second pair.

The vias 122, 182 of the second electrode 120 and the additional second electrodes 180 may be utilized additionally as the leads for a resistive heater 190. This is possible, even without stopping the current to the heater 190 during the measurements of the electrode pairs, because the electrolyte impedance between electrodes of the pairs is measured based on an alternating current (AC) signal, while the heater 190 can be operated using a direct current (DC) signal, or as an AC signal with a different frequency.

The difference between the two measurements by the first pair of electrodes and the second pair of electrodes, i.e., the measured resistance values, the mass flow rate of the fluid can be determined (flowing in the direction between the two electrode pairs).

The electrolyte conductivity of the fluid has a temperature dependence (via fluid viscosity) according to the Arrhenius equation. Thus, the electrolyte conductivity is proportional to *e*^{*-Ea*/*kT*}, where *Eₐ* is an apparent activation energy associated with intermolecular forces between fluid molecules, k is the Boltzmann constant, and T is absolute temperature. For (acqueous) fluids, this can be approximated to 1.02^{(*T-T*0)}*,* where *T*₀ is an original temperature before heating, since *Eₐ*~2.8 · 10⁻²⁰*J* >> *kT.* Thus, the fluid is provided with a 2% higher conductivity per degree of temperature rise.

Taking a ratio of measurements, the temperature independent pre-factors for upstream and downstream electrolyte conductivities cancel out. Then, ratio of downstream conductivity over upstream conductivity becomes equal to 1.02^{(*Tdown-Tup*)}, where *T_{down}* is a temperature at a downstream position and *Tᵤₚ* is a temperature at an upstream position in relation to flow of fluid.

Thus, the temperature difference between downstream and upstream positions (Δ*T* = *T_{down}* - *Tᵤₚ*) can be determined from two measurements of electrolyte conductivity by the first and second electrode pairs. According to the calorimetric flow measurement principle, this may be used to determine the local flow rate.

Thanks to the use of the electrochemical sensor arrangement 100 having rotational symmetry of the electrode pairs, highly accurate determination of the fluid conductivity may be performed. Moreover, fluid conductivity strongly depends on fluid temperature: forementioned 2%/°C, which is much more sensitive than a conventional Pt-100 thermistor: 0.3%/°C at 37°C.

The electrochemical sensor arrangement 100 may be configured to provide thermal conductance of a material below the heater 190 having a lower value than the thermal conductance of fluid above the heater 190. This ensures that heat flux from the heater 190 is predominantly used to heat the fluid, not the material below the heater 190. This will improve the accuracy and sensitivity of the mass flow sensor.

The electrochemical sensor arrangement 100 may thus comprise a layer 192 of thermally insulating material and the heater 190 may be arranged on the layer 192. Measures to reduce the thermal conductance of the substrate on which the heater 190 is arranged may involve using silicon oxide as a top layer on a silicon substrate, or alternatively using a glass substrate. As a further alternative, a photoresist layer 192 may be deposited on top of the substrate on which the resistive heater 190 is patterned. Additionally, reducing the thickness of the substrate is beneficial, for instance by thinning the substrate thickness locally below the resistive heater 190, by etching a glass substrate from a bottom side, optionally completely, leaving only a photoresist membrane on which the heater 190 is located.

Finally, an additional benefit of determining the fluid temperature difference from the fluid conductivity ratio, is that there is no need to add thermally insulating material below the electrode pairs, since these measure not the temperature of the electrodes itself but of the fluid above it. This approach gives better sensitivity than thermistors (e.g. Pt-100) which measure the temperature of electrode itself, and will therefore be more affected by thermal conductivity of the underlying substrate.

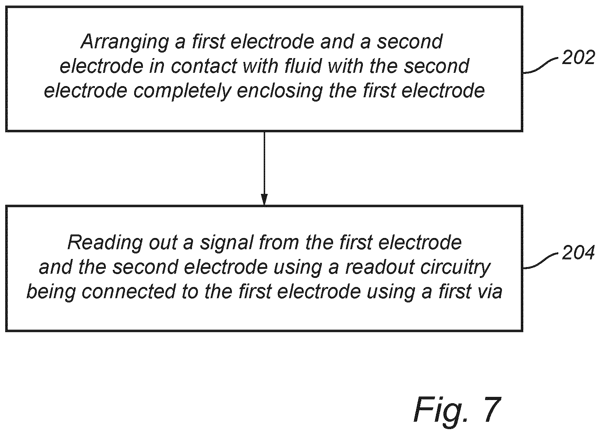

Referring now to Fig. 7, a method for measuring a property of a fluid will be described.

The method comprises arranging 202 a first electrode and a second electrode in contact with the fluid, wherein the first electrode and the second electrode are arranged on a first surface of a first layer. The first electrode has a first shape on the surface and the second electrode has a second shape on the surface. The second shape of the second electrode defines an opening within the second electrode such that the second shape of the second electrode completely encloses the first shape of the first electrode with a spacing being defined between the first electrode and the second electrode.

The method further comprises reading out 204 a signal from the first and second electrodes by a readout circuitry. The readout circuitry is formed at a second surface of the first layer opposite to the first surface or at a substrate separate from the first layer. Further, a first connection between the first electrode and the readout circuitry comprises a first via extending from the first shape of the first electrode through the first layer.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. An electrochemical sensor arrangement (100) for measuring a property of a fluid, said electrochemical sensor arrangement (100) comprising:
a first electrode (110) and a second electrode (120) arranged on a first surface (132) of a first layer (130), wherein the first electrode (110) and the second electrode (120) are configured to make contact with the fluid for measuring the property of the fluid; and
readout circuitry (140) connected to the first electrode (110) and the second electrode (120) for reading out signals from the first and second electrodes, wherein the readout circuitry (140) is formed at a second surface (134) of the first layer (130) opposite to the first surface (132) or at a substrate (142) separate from the first layer (130);
wherein the first electrode (110) has a first shape on the first surface (132) and the second electrode (120) has a second shape on the first surface (132), wherein the second shape of the second electrode (120) defines an opening within the second electrode (120) such that the second shape of the second electrode (120) completely encloses the first shape of the first electrode (110) with a spacing (114) being defined between the first electrode (110) and the second electrode (120); and
wherein a first connection (136) between the first electrode (110) and the readout circuitry (140) comprises a first via (112) extending from the first shape of the first electrode (110) through the first layer (130).

2. The electrochemical sensor arrangement according to claim 1, wherein an outer border of the opening defined by the second shape of the second electrode (120) has a corresponding shape to the first shape of the first electrode (110) such that the spacing (114) between the first electrode (110) and the second electrode (120) is equidistant across the first shape of the first electrode (110).

3. The electrochemical sensor arrangement according to claim 2, wherein the outer border of the opening and an outer border of the first shape form concentric circles.

4. The electrochemical sensor arrangement according to any one of the preceding claims, wherein a second connection (138) between the second electrode (120) and the readout circuitry (140) comprises a second via (122) extending from the second shape of the second electrode (120) through the first layer (130).

5. The electrochemical sensor arrangement according to any one of the preceding claims, wherein the read-out circuitry (140) is formed at the substrate (142) separate from the first layer (130), wherein the first connection (136) includes a contact point (144) formed at the second surface (134) of the first layer (130), wherein the first via (112) is configured to extend from the first shape of the first electrode (110) to the contact point (144), wherein the first connection (136) is further configured to connect the contact point (144) and the readout circuitry (140) being arranged at the substrate (142) being bonded to the first layer (130).

6. The electrochemical sensor arrangement according to any one of the preceding claims, wherein the first layer (130) is a substrate formed by silicon or glass.

7. The electrochemical sensor arrangement according to any one of the preceding claims, wherein the first via (112) at the first surface (132) of the first layer (130) has a smaller cross-section than the first shape of the first electrode (110) and the first via (112) is fully covered by the first shape of the first electrode (110).

8. The electrochemical sensor arrangement according to any one of the preceding claims, wherein the first via (112) has a length of at least 20 µm, such as at least 100 µm, extending through the first layer (130).

9. The electrochemical sensor arrangement according to any one of the preceding claims, wherein at least a part of the first shape of the first electrode (110) is provided with a bioreceptor (116) configured to interact with an analyte in the fluid.

10. The electrochemical sensor arrangement according to any one of the preceding claims, further comprising a reference electrode (150) arranged on the first surface (132) of the first layer (130), wherein a connection between the reference electrode (150) and the readout circuitry (140) comprises a reference via (152) extending from the reference electrode (150) through the first layer (130).

11. The electrochemical sensor arrangement according to any one of the preceding claims, further comprising an additional electrode (160), wherein the first electrode (110) and the additional electrode (160) are configured for performing separate measurements of the property of the fluid, wherein the second electrode (120) is configured to provide a shared counter electrode to the first electrode (110) and the additional electrode (160), wherein the second shape of the second electrode (120) defines separate openings for the first electrode (110) and the additional electrode (160) within the second electrode (120) such that the second shape of the second electrode (120) further completely encloses a shape of the additional electrode (160) with a spacing (164) being defined between the additional electrode (160) and the second electrode (120).

12. The electrochemical sensor arrangement according to any one of claims 1-10, wherein the first electrode (110) and the second electrode (120) form a first pair of electrodes and the electrochemical sensor arrangement (100) further comprises:
a second pair of electrodes comprising an additional first electrode (170) and an additional second electrode (180), wherein a shape of the additional second electrode (180) defines an opening within the additional second electrode (180) such that the shape of the additional second electrode (180) completely encloses a shape of the additional first electrode (170) with a spacing (174) being defined between the additional first electrode (170) and the additional second electrode (180), wherein an additional first connection between the additional first electrode (170) and the readout circuitry (140) comprises an additional first via (172) extending from the shape of the additional first electrode (170) through the first layer (130); and
a heater (190) arranged between the first pair of electrodes and the second pair of electrodes;
wherein the first pair of electrodes is configured to perform a first measurement of the property of the fluid and the second pair of electrodes is configured to perform a second measurement of the property of the fluid, wherein a difference between the first measurement and the second measurement is representative of mass flow of the fluid.

13. The electrochemical sensor arrangement according to claim 12, wherein the heater (190) is arranged on a thermally insulating material (192).

14. A method for measuring a property of a fluid, said method comprising:
arranging (202) a first electrode and a second electrode in contact with the fluid, wherein the first electrode and the second electrode are arranged on a first surface of a first layer, wherein the first electrode has a first shape on the surface and the second electrode has a second shape on the surface, wherein the second shape of the second electrode defines an opening within the second electrode such that the second shape of the second electrode completely encloses the first shape of the first electrode with a spacing being defined between the first electrode and the second electrode; and
reading out (204) a signal from the first and second electrodes by a readout circuitry, wherein the readout circuitry is formed at a second surface of the first layer opposite to the first surface or at a substrate separate from the first layer, and wherein a first connection between the first electrode and the readout circuitry comprises a first via extending from the first shape of the first electrode through the first layer.
